# EUROPÄISCHE PATENTSCHRIFT

(11) **EP 1 478 929 B1**
(45) Veröffentlichungstag und Bekanntmachung des Hinweises auf die Patenterteilung: **06.04.2011**
(21) Anmeldenummer: 03709720.1
(22) Anmeldetag: 25.02.2003
(51) Int. Cl.: G01N 33/68

(54) **VERFAHREN ZUM NACHWEIS VON ERKRANKUNGEN, DIE AUF DEFEKTEN DES PROTEINS CYSTIC FIBROSIS TRANSMEMBRANE CONDUCTANCE REGULATOR (CFTR) BERUHEN**
METHOD FOR DETECTING DISEASES THAT ARE ASSOCIATED WITH DEFECTS OF CYSTIC FIBROSIS TRANSMEMBRANE CONDUCTANCE REGULATOR (CFTR) PROTEIN
PROCEDE DE DETECTION DE PATHOLOGIES QUI REPOSENT SUR DES DEFICIENCES DE LA PROTEINE CFTR (REGULATEUR DE LA CONDUCTANCE TRANSMEMBRANAIRE DE LA MUCOVISCIDOSE)

(30) Priorität: 26.02.2002 DE 10208293; 12.04.2002 DE 10216160
(43) Veröffentlichungstag der Anmeldung: 24.11.2004
(73) Patentinhaber: Schillers, Hermann, 48155 Münster (DE); Oberleithner, Hans, 48149 Münster (DE)
(72) Erfinder: Schillers, Hermann, 48155 Münster (DE); Oberleithner, Hans, 48149 Münster (DE)
(74) Vertreter: Von Renesse, Dorothea
(86) Internationale Anmeldenummer: PCT/EP2003/001904
(87) Internationale Veröffentlichungsnummer: WO 2003/073103

(56) Entgegenhaltungen:
- WO-A-94/15218
- BRONSVELD INEZ ET AL: "Chloride conductance and genetic background modulate the cystic fibrosis phenotype of DELTAF508 homozygous twins and siblings." JOURNAL OF CLINICAL INVESTIGATION, Bd. 108, Nr. 11, Dezember 2001 (2001-12), Seiten 1705-1715, XP002243563 December, 2001 ISSN: 0021-9738
- SULEYMANIAN MIRIK A ET AL: "Stretch-activated channel blockers modulate cell volume in cardiac ventricular myocytes." JOURNAL OF MOLECULAR AND CELLULAR CARDIOLOGY, Bd. 27, Nr. 1, 1995, Seiten 721-728, XP009011385 ISSN: 0022-2828
- REDDY M M ET AL: "Effect of anion transport blockers on CFTR in the human sweat duct." JOURNAL OF MEMBRANE BIOLOGY, Bd. 189, Nr. 1, 1. September 2002 (2002-09-01), Seiten 15-25, XP009011395 ISSN: 0022-2631
- BRAUNSTEIN GAVIN M ET AL: "Cystic fibrosis transmembrane conductance regulator facilitates ATP release by stimulating a separate ATP release channel for autocrine control of cell volume regulation." JOURNAL OF BIOLOGICAL CHEMISTRY, Bd. 276, Nr. 9, 2. März 2001 (2001-03-02), Seiten 6621-6630, XP001157417 ISSN: 0021-9258 in der Anmeldung erwähnt

## Beschreibung

Die Erfindung betrifft nicht-genetische Nachweisverfahren und -mittel für Erkrankungen, die auf Defekte des CFTR beruhen, insbesondere für die Erkrankung der Mukoviszidose resp. zystischen Fibrose (engl.: cystic fibrosis oder CF) und nimmt die Priorität der deutschen Patentanmeldungen 102 08 293.6 und 102 16 160.7 in Anspruch, auf die inhaltlich Bezug genommen wird.

Verschiedene auf Defekte des CFTR basierende Krankheiten, wie beispielsweise die zystische Fibrose, das kongenitale Fehlen des Vas Deferenz und einige Formen der Pankreatitis sind bekannt. Die zystische Fibrose - auch Mukoviszidose genannt - stellt eine der am häufigsten vorkommenden genetisch-bedingten Erkrankungen dar. Die Krankheit tritt in regional unterschiedlicher Häufigkeit bei ca. 1 : 2500 der Neugeborenen auf.

Mukoviszidose wird autosomal rezessiv vererbt und geht auf einen Defekt am langen Arm des Chromosoms 7 zurück. Betroffen ist das Gen, das für das Protein CFTR (cystic fibrosis transmembrane conductance regulator), ein Membrantransportprotein kodiert. Das Hauptmerkmal dieser schwerwiegenden Erkrankung ist eine allgemeine Fehlfunktion von Epithelien, aller exokrinen Drüsen, der Lunge und des Verdauungstraktes. Die Krankheit äußert sich durch eine erhöhte Viskosität des Sekrets der mukösen Drüsen in der Lunge und in der Bauchspeicheldrüse. Im fortschreitenden Krankheitsverlauf kommt es zu erheblichen anatomischen Veränderungen, die schwere Komplikationen im Bereich der Atemwege, wie zum Beispiel chronische Infektionen mit Emphysem der Lunge und schwerwiegende Störungen der Verdauung (Malabsorption) mit Flüssigkeits- und Elektrolytverlusten zur Folge haben. Die Behandlung der zystischen Fibrose erfolgt durch Enzymsubstitution, gezielte Antibiotikagaben und physiotherapeutische Behandlung (Klopfmassage). Die Krankheit ist nicht heilbar. Mukoviszidose-Kranke leiden unter einer erheblichen Einschränkung ihrer Lebensqualität und erreichen trotz der heute guten medizinischen Betreuung in der Regel nur ein Lebensalter von ca. 40 Jahren.

Die Krankheitsentstehung ist noch nicht eindeutig geklärt. Das betroffene Gen kodiert für einen in der Membran vorwiegend epithelialer Zellen lokalisierten Chlorid-Kanal. Eine Vielzahl unterschiedlicher Mutationen, die zu erhöhter Schleimviskosität und einer veränderten Zusammensetzung der Sekrete führen, ist beschrieben (Pschyrembel, Klinisches Wörterbuch, 258. Auflage). Die häufigste Mutation ist die Deletion von Phenylalanin an Position 508 (ΔF508). Neben der homozygoten Form der Mukoviszidose kann sich die Krankheit auch bei heterozygoten Genträgern mit unterschiedlichem Schweregrad und auch noch im fortgeschrittenen Lebensalter entwickeln. Etwa 4 % der weißen Bevölkerung Europas und der USA tragen eine heterozygote CFTR-Mutation.

Aus der Literatur ist bekannt, daß bei CF-Patienten mit der homozygoten Mutation ΔF508 das CFTR-Protein die Plasmamembran der Zellen entweder nicht erreicht oder nur in unzureichendem Maße in der Membran verankert wird. Ein solches CFTR-Protein ist dann funktionsunfähig.

Aus der Literatur ist weiterhin bekannt, daß der CFTR einen erheblichen Einfluß auf die Regulation des Zellvolumens hat. In Zellen, in denen die CFTR-Funktion nicht gestört ist, greift dieser in einen autokrinen Mechanismus ein, der durch die Freisetzung von und die Signalübertragung durch ATP (Adenosintriphosphat) über einen separaten Ionenkanal gesteuert wird. Ein hypotones Medium bewirkt einen zelleinwärts gerichteten Wassereinstrom. Die daraus resultierende Zellschwellung aktiviert den CFTR, der wiederum einen ATP-Transport aus der Zelle heraus auslöst. Das extrazelluläre ATP aktiviert purinerge Rezeptoren, die in der Zelle die Phospolipase C (PLC) zur Bildung von Inositoltriphosphat (IP₃) anregen. Das Inositoltriphosphat erhöht die intrazelluläre Konzentration an Kalziumionen (Ca²⁺) . Eine erhöhte intrazelluläre Kalziumionenkonzentration aktiviert die kalziumabhängigen Kalium- und Chlorid-Kanäle sowie Kanäle für Osmolyte, wie beispielsweise Taurin. Daraufhin verlassen Kalium- und Chloridionen die Zelle. Durch diesen Nettosalzverlust strömt Wasser osmotisch hinterher. In der Folge schrumpft die Zelle (Braunstein, G. B. et al., The Journal of Biological Chemistry, Vol. 276, No. 9, Issue of March 2, pp. 6621-6630, 2001). Dieser Vorgang wird als regulierte Volumenverminderung, engl.: regulatory volume decrease (RVD) bezeichnet. Es wurde gezeigt, dass humane rote Blutzellen nach mechanischer Deformation CFTR-abh. ATP freisetzen (Sprague RS, Ellsworth ML, Stephenson AH, Kleinhenz ME, Lonigro AJ, Deformation-induced ATP release from red blood cell requires cystic fibrosis transmembrane conductance regulator activity. American Journal of Physiology, 275:H1726-H1732, 1998). Es wurde gezeigt, dass in hypotonen Medien die roten Blutzellen einiger Spezies ihr Volumen ATP abhängig regulieren, humane rote Blutzellen jedoch nicht. (Light DB, Capes TL, Gronau RT, Adler MR. Extracellular ATP stimulates volume decrease in Necturus red blood cell. American Journal of Physiology, Sep;277(3 Pt 1):C480-91, 1999).

Methoden der CF-Diagnostik:
1) Bislang erfolgt die Diagnose der zystischen Fibrose unter Anderem aufgrund einer Schweißuntersuchung, bei der die CFTR-abhängige Chlorid-Resorption der Epithelzellen des Schweißganges durch Bestimmung der Chlorid-Konzentration im Schweiß ermittelt wird (Patent-Nr.: WO00/13713 Titel: Macroscopic sweat test for cystic fibrosis). Bei CF-Patienten wird eine erhöhte Chlorid-Konzentration gefunden. Der Schweißtest liefert häufig unklare Ergebnisse. Er ist zudem sehr zeit- und personalaufwendig und somit teuer.
2) Weitere Methoden sind die Messung des durch die Pankreasinsuffizienz bei CF-Patienten in das Blut gelangten immunreaktiven Trypsins (Patent-Nr.: AU 6445186, Titel: Detection of immunreactive trypsin and cystic fibrosis using monoclonal antibody) und
3) der Nachweis einer Erhöhung des Albumingehaltes im Mekonium (Kindspech) von Säuglingen (Patent-Nr.: US 3,902,847, Titel: Diagnostic device and method for the diagnosis of mucoviscidosis (cystic fibrosis)).
4) Darüber hinaus kann die CF-Diagnose über die Untersuchung des Speicheldrüsensekrets und durch
5) die zwar genaue, aber aufwendige und damit bisher wenig angewandte transepitheliale Potentialmessung an der Nasenschleimhaut vorgenommen werden.
6) Eine weitere Möglichkeit der Diagnose der zystischen Fibrose besteht in der Erkennung der Mutation über die direkte Genanalyse (Patent-Nr.:
   WO94/15218, Titel: Detection of cystic fibrosis or a gene mutation). Die genetische Untersuchung läßt allerdings nur sehr begrenzt auf funktionelle Störungen rückschließen. Es sind bisher viele hundert verschiedene CFTR-Mutationen bekannt, die zu mehr oder minder ausgebildeten klinischen Bildern führen. In den genetischen Analysen wird jedoch nur auf ca. 15 verschiedene Mutationen untersucht, die nur etwa 80 % aller CF-Erkrankungen hervorrufen.
7) Es wurde ein CF-Test angeregt, bei dem durch mechanische Deformation von Erythrozyten freigesetztes ATP mit dem Luziferin-Luziferase-Assay gemessen werden soll. Dieses basiert auf der Beobachtung einer Beziehung zwischen CFTR und ATP-Sekretion. (Verloo, P. et al., Pediatric Pulmonology, Suppl. 20:72 (2000)).
8) Weitere Methoden basieren auf Unterschiede zwischen CF und nicht-CF in der Kinetik einiger Enzyme, wie z.B. der NADH-Dehydrogenase der Mitochondrien (Patent-Nr.: PCT/US80/00370, Titel: Cystic fibrosis detection method), die z.B. im Lymphozytenhomogenisat gemessen werden kann.
9) Das Enzym Hydrolase von CF-Patienten zeigt unter bestimmten Testbedingungen eine schnellere Inaktivierungskinetik als die Hydrolase von gesunden Probanden (Patent-Nr.: US 4,469,788, Titel: In vitro diagnosis of cycstic fibrosis).

Die bekannten Nachweisverfahren sind z.T. teuer und zeigen den Nachteil, daß sie wenig zuverlässig und somit kein zuverlässiges Neugeborenen-Screening auf Mukoviszidose erlauben. Die genetische Untersuchung ist bei Neugeborenen möglich, erlaubt aber bei heterozygoter Mutation keine Aussage über das funktionelle Ausmaß des CFTR-Defektes. Der Schweißtest ist mit erheblichen Schwankungen verbunden. Die Messung des immunreaktiven Trypsins und die Messung des Albumins im Mekonium sind wenig verläßlich.

Durch eine möglichst frühzeitige - am effektivsten beim Neugeborenen - durchgeführte verläßliche Diagnose der zystischen Fibrose könnte eine zielgerichtete Behandlung ab dem ersten Lebenstag die gesundheitliche Situation der Patienten verbessern und damit die Lebenserwartung deutlich erhöhen, da sich in diesem Fall die schwerwiegenden, krankheitsbedingten anatomischen Veränderungen weniger stark ausprägen würden.

Da die Standardverfahren keine sichere frühzeitige Diagnose der zystischen Fibrose erlauben, können CF-Erkrankungen aber erst zu einem späten Zeitpunkt sicher erkannt werden. In der klinischen Praxis werden Säuglinge in begründeten Verdachtsfällen insbesondere mit Antibiotika behandelt, was Kosten verursacht und zu gesundheitlichen Beeinträchtigungen führen kann. Bei den heterozygoten CF-Mutationen kann sich das Krankheitsbild in den verschiedenen Schweregraden erst in der Jugend oder noch später im Erwachsenenalter manifestieren und ist somit einer erfolgreichen Frühbehandlung nicht zugänglich. Des weiteren existieren Krankheitsbilder, wie zum Beispiel bestimmte Pankreas-Erkrankungen, die mit Mutationen des CFTR-Gens in Zusammenhang gebracht werden.

Der Erfindung liegt nun die Aufgabe zugrunde, eine geeignete Methode zur Verfügung zu stellen, die Erkrankungen, die auf Defekten des CFTR beruhen, insbesondere die zystische Fibrose, zu einem möglichst frühen Zeitpunkt zuverlässig nachzuweisen.

Die Aufgabe wird gelöst durch ein Nachweisverfahren gemäß Anspruch 1. Die Bestimmung erfolgt durch die Destabilisierung der CFTR abhängigen Volumenregulation in Blutzellen und die anschließende Diskriminierung zwischen CFTR-Defekten und CFTR-intakten Zellen. Diese ist möglich durch die Darstellung der Zell-Lyse der CFTR-intakten Zellen.

Eine nicht defekte Zelle ist in der Lage, unter physiologischen Bedingungen ihr Zellvolumen durch ineinandergreifende Mechanismen über den Einstrom und den Ausstrom von Ionen und Wasser zu kontrollieren und zu steuern. In nicht defekten Blutzellen strömt Chlorid nach Einbringen in ein 155 mM Kaliumchlorid-Medium unter anderem über den funktionsfähigen CFTR in die Zelle. Der Erfindung liegt nun der Gedanke zugrunde, durch die Beeinflussung, z. B. Blockade, in der Zellmembran exprimierter, CFTR-abhängiger ATP-transportierender Kanäle die regulierte Volumenverminderung (RVD) zu verhindern. Einströmende Kalium- und Chloridionen akkumulieren in der funktionsfähigen nicht defekten Zelle, was durch den damit einhergehenden Wassereinstrom zur Lyse der Blutzellen führt, während die Lyse bei defekten Blutzellen, denen es bereits an dem Einwärtstransport der Ionen fehlt, ausbleibt. Dies ist insbesondere für CF-Blutzellen der Fall, bei denen durch den CFTR-Defekt keine Chlorid-Ionen über den CFTR in die Blutzellen einströmen und deren RVD CFTR-unabhängig ist. Infolgedessen kann sich in der Zelle auch kein Überdruck aufbauen; die Blutzellen mit defekten Ionenkanälen, insbesondere die Blutzellen von CF-Patienten unterliegen keiner Lyse.

Aus dem Stand der Technik ist bereits bekannt, daß ein milder CF-Phänotyp mit der Funktionalität von CFTR-Kanälen korreliert und diese Korrelation über eine Cl⁻-Sekretion nachweisbar ist. (Bronsveld Inez et al.: "Chloride conductance and genetic background modulate the cystic fibrosis phenotype of ΔF508 homozygous twins and siblings, Journal of Clinical Investigation, vol. 108, No. 11, Dezember 2001, 1705-1715). Ebenso ist bekannt, daß die mit CFTR-Defekten einhergehende gestörte regulierte Volumenverminderung zur Pathogenese der zystischen Fibrose beiträgt (Reddy M. M. et al. "Effect on Anion Transport Blockers on CFTR in the Human Sweat Duct, Journal of Membrane Biology, . 189, 2002, 15-25). Es wurde bislang jedoch noch nicht erkannt, daß dieser Zusammenhang auch in Blutzellen existiert und durch das Ermitteln des Vorhandenseins von Blutzellen mit abweichender Volumenänderung zur Bestimmung von auf CFTR-Defekten beruhenden Erkrankungen genutzt werden kann.

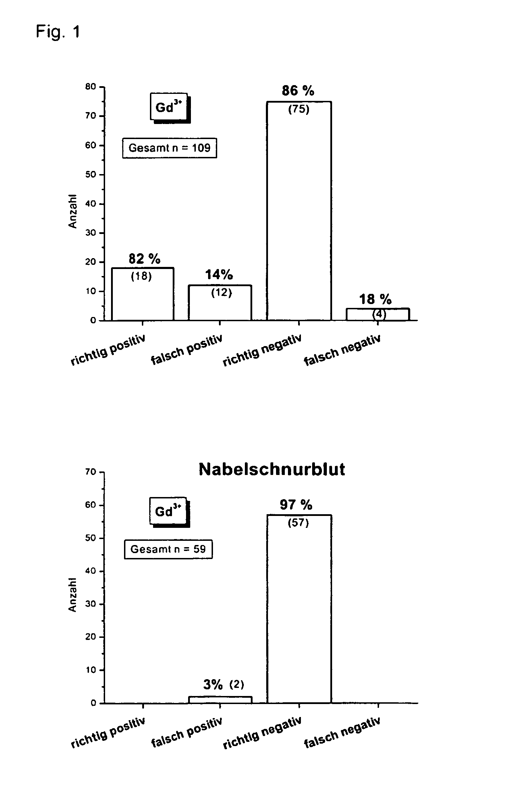

Bei den Mukoviszidose-Kranken mit einer homozygoten ΔF508 Mutation befindet sich das CFTR nicht in der Zellmembran. Bei heterozygoten Mutationen und anderen Mutationen als ΔF508 können sich kleine Mengen funktionsfähigen CFTR in der Membran befinden, die Chlorid transportieren und an der Volumenregulation beteiligt sind. Dabei ist die Menge der in der Zellmembran vorhandenen funktionsfähigen CFTR-Moleküle umgekehrt proportional zum Schweregrad der Erkrankung. Durch die Blockade der CFTR-ab-Untersuchungen des Blutes von gesunden Personen, Mukoviszidose-Patienten und postnatal aus der Nabelschnur entnommenen Blutproben von Neugeborenen (Nabelschnur-Blut) bestätigen die einfache Durchführbarkeit und die gute Aussagekraft des erfindungsgemäßen Testverfahrens, bei dem Gd³⁺ zur Blockade der Volumenregulation eingesetzt wird. Die statistische Auswertung der bisherigen Untersuchungsergebnisse mit 22 CF-Patienten und 87 gesunden Probanden sowie 59 Neugeborenen (Nabelschnur-Blut) zeigt eine eindeutige Zunahme des Hämolysegrades nach Zugabe von Gd³⁺ bei den gesunden Probanden und beim Nabelschnur-Blut. Bei den CF-Patienten wird eine nur geringfügige Zunahme des Hämolysegrades nach Gd³⁺-Applikation verzeichnet. Von den 87 gesunden Probanden wurden 75 als nicht-CF erkannt, d.h. richtig negativ (86%), und 12 wurden als CF erkannt, d.h. falsch positiv (14%). Von den 22 CF-Patienten wurden 18 als CF erkannt, d.h. richtig positiv (82%), und 4 wurden als nicht-CF erkannt, d.h. falsch negativ (18%). Von den 59 Neugeborenen wurden 57 als nicht-CF erkannt, d.h. richtig negativ (97%), und 2 wurden als CF erkannt, d.h. falsch positiv (3%) (Fig. 1).

Beispiel für ein Nachweisverfahren gemäß Anspruch 3 für Blutzellen:
Die Aufgabe wird gelöst durch ein Nachweisverfahren gemäß Anspruch 3, das insbesondere geeignet ist, auf einen Defekt des CFTR beruhende Krankheiten durch eine auf einer Zelllyse nicht defekter Blutzellen basierende Ermittlung von defekten Blutzellen durch Aktivierung (Öffnung) von bestimmten Ionenkanälen der nicht defekten Blutzellen zu diagnostizieren.

Der Erfindung gemäß Anspruch 3 liegt nun der Gedanke zugrunde, insbesondere durch Aktivierung von bestimmten CFTR-abhängigen Ionenkanälen in nicht defekten Blutzellen, durch die Anionen wie z. B.: Iodid (I⁻), Bromid (Br⁻), Chlorid (Cl⁻), Fluorid (F), Gluconat (Gluconsäure-Anion), Rhodanid (SCN⁻) und Taurin in die nicht defekten Blutzellen einströmen, eine Lyse der nicht defekten Blutzellen hervorzurufen. In defekten Blutzellen sind diese CFTR-abhängigen Ionenkanäle nicht vorhanden oder nicht aktivierbar. Deshalb kommt es nicht zum Einstrom von Anionen wie z. B.: Iodid, Bromid, Chlorid, Fluorid, Gluconat, Rhodanid und Taurin in die defekte Zelle. Infolgedessen kann sich in der Zelle auch kein Überdruck aufbauen; die Blutzellen mit defekten CFTR, insbesondere die Blutzellen von CF-Patienten unterliegen keiner Lyse.

Da dieser CFTR-abhängige Ionenkanal in nicht defekten Blutzellen Anionen wie z. B.: Iodid, Bromid, Chlorid, Fluorid, Gluconat und Rhodanid sowie Taurin durchläßt, kann die Unterscheidung zwischen defekten und nicht defekten Blutzellen auch in 155 mM Lösungen erfolgen, die als Anion Iodid, Bromid, Fluorid, Gluconat, Rhodanid oder Taurin enthalten.

Die Blutzellen mit defekten Ionenkanälen, insbesondere die Blutzellen von CF-Patienten unterliegen keiner Lyse.

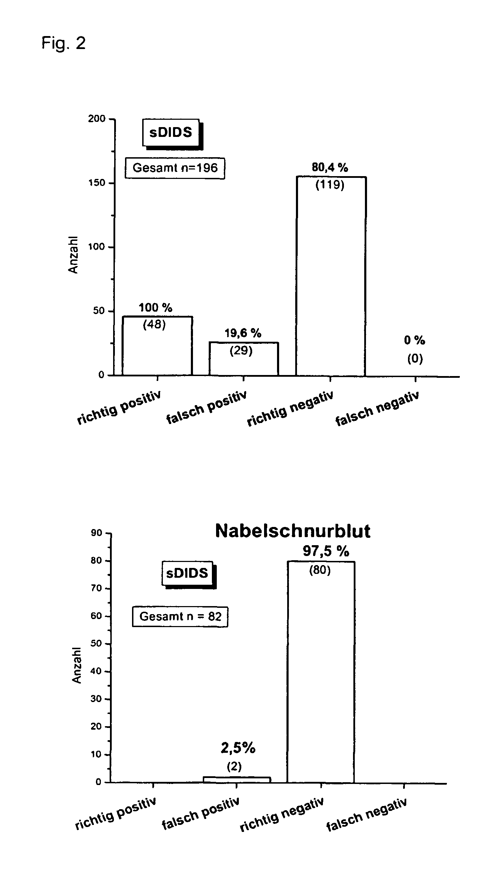

Die statistische Auswertung der Untersuchungsergebnisse mit 48 CF-Patienten und 148 gesunden Probanden sowie 82 Neugeborenen (Nabelschnur-Blut), zeigt eine eindeutige Zunahme des Hämolysegrades nach Zugabe von sDIDS (4-Amino-4'-Isothiocyanato-Stilben-2,2'-Disulfonsäure wird im folgenden sDIDS genannt) bei den gesunden Probanden und beim Nabelschnur-Blut. Bei den CF-Patienten kann eine nur geringfügige Zunahme des Hämolysegrades nach sDIDS-Applikation verzeichnet werden. Von den 148 gesunden Probanden wurden 119 als nicht-CF erkannt, d.h. richtig negativ (80,4%), und 29 wurden als CF erkannt, d.h. falsch positiv (19,6%). Von den 48 CF-Patienten wurden alle 48 als CF erkannt, d.h. richtig positiv (100%). Von den 82 Neugeborenen wurden 80 als nicht-CF erkannt, d.h. richtig negativ (97,5%), und 2 wurden als CF erkannt, d.h. falsch positiv (2,5%) (Fig. 2).

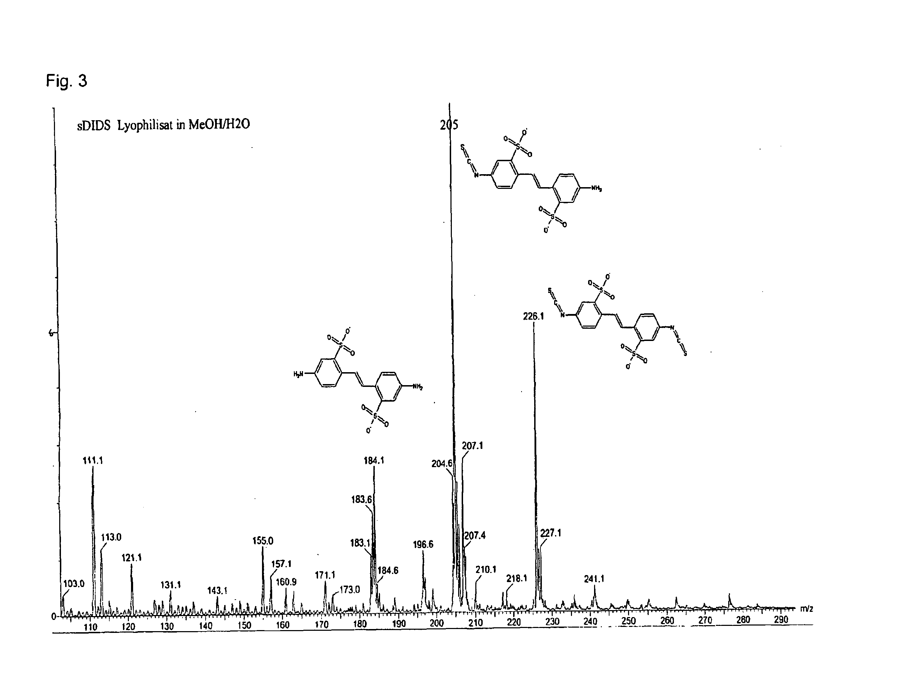

Die Aktivierung eines bestimmten CFTR-abhängigen Ionenkanals, der Anionen wie z. B.: Iodid, Bromid, Chlorid, Fluorid, Gluconat, Rhodanid und Taurin durchläßt, gelingt mit einer Substanz, die durch besondere Behandlung von DIDS (4,4'-Diisothiocyanato-Stilben-2,2'-Disulfonsäure, Natriumsalz) entsteht. DIDS wird in DMSO gelöst (0, 1 M) und für 4 Wochen bei 4°C im Kühlschrank gelagert. Durch diese Lagerung von DIDS entsteht ein Hydrolyseprodukt von DIDS durch in DMSO enthaltenem Restwasser. Untersuchungen dieser Lösung mit massenspektrometrischen Methoden zeigen, dass neben DIDS auch 4,4'-Diamino-Stilben-2,2'-Disulfonsäure (DADS) und 4-Amino-4'-Isothiocyanato-Stilben-2,2'-Disulfonsäure in der Lösung vorhanden sind (Fig. 3).

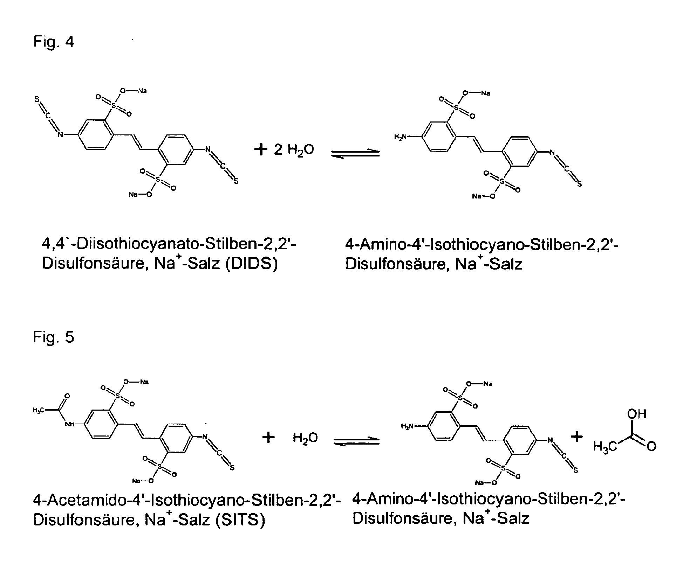

Durch partielle Hydrolyse von DIDS entsteht die 4-Amino-4'-Isothiocyanato-Stilben-2,2'-Disulfonsäure, H₂S und CO₂ (Fig. 4):
4,4'-Diisothiocyanato-Stilben-2,2'-Disulfonsäure, Na⁺-Salz (DIDS) + 2H₂O => 4-Amino-4'-Isothiocyanato-Stilben-2,2'-Disulfonsäure, Na⁺-Salz + H₂S + CO₂.

Ebenso führt eine Lagerung von 4-Acetamido-4'-Isothiocyanato-Stilben-2,2'-Disulfonsäure (SITS) in wasserhaltigem DMSO bei 4°C über Wochen zu Hydrolyseprodukten, so dass in einer solchen Lösung neben SITS auch DADS und 4-Amino-4'-Isothiocyanato-Stilben-2,2'-Disulfonsäure zu finden sind. Durch partielle Hydrolyse von SITS entsteht die 4-Amino-4'-Isothiocyanato-Stilben-2,2'-Disulfonsäure und Essigsäure (Fig. 5):
4-Acetamido-4'-Isothiocyanato-Stilben-2,2'-Disulfonsäure, (SITS) +H₂O => 4-Amino-4'-Isothiocyanato-Stilben-2,2'-Disulfonsäure + Essigsäure 4-Amino-4'-Isothiocyanato-Stilben-2,2'-Disulfonsäure wird für die vorliegende Erfindung als sDIDS bezeichnet.

Auch das Produkt der partiellen Hydrolyse von 4,4'-Diisothiocyanato-Dihydrostilben-2,2'-disulfonsäure, Natriumsalz (H₂DIDS) wirkt wie sDIDS. Es unterscheidet sich von sDIDS nur durch eine Einfachbindung der Kohlenstoffatome zwischen den Phenylringen im Gegensatz zu einer Doppelbindung bei sDIDS.
4,4'-Diisothiocyanato-Dihydrostilben-2,2'-disulfonsäure, Na⁺-Salz (H₂DIDS) + 2H₂O
4-Amino-4'-Isothiocyanato-Dihydrostilben-2,2'-disulfonsäure, Na⁺-Salz + H₂S + CO₂

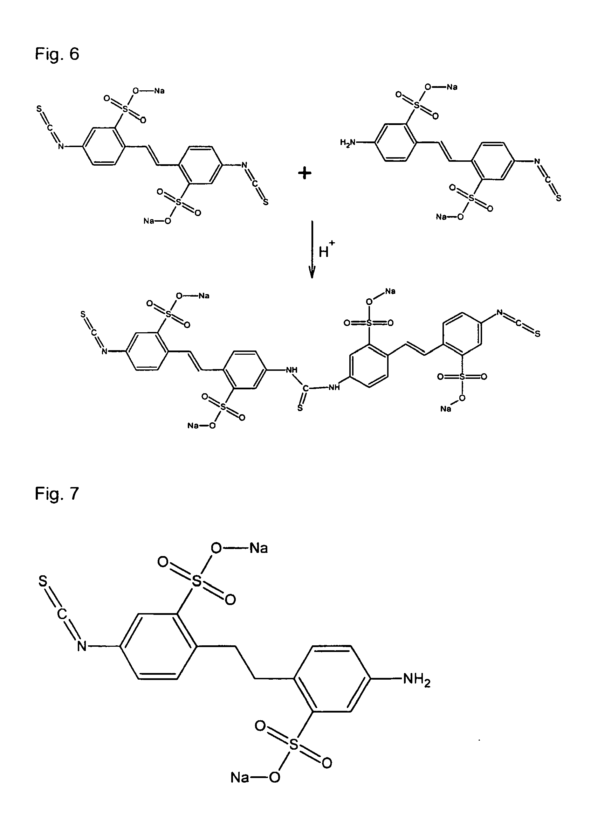

Die Strukturformel ist in Fig. 7 gezeigt.

Bereits 1987 wurde von Horobin, Payne und Jakobsen (Horobin RW, Payne JN, Jakobsen P, Histochemical implications of the biological properties of SITS and some related compounds. Journal of Microscopy, 1987 Apr; 146(1):87-96) beschrieben, dass sich SITS und DIDS in wässriger Lösung durch Hydrolyse der Acetamido-Gruppe bzw. einer Isothiocyanat-Gruppe zu 4-Amino-4'-Isothiocyanato-Stilben-2,2'-Disulfonsäure umsetzen. Dieses geschieht bei Raumtemperatur in einer Woche. Nach drei Wochen hat sich durch Hydrolyse der verbleibenden Isothiocyanat-Gruppe das DADS gebildet. Bei Lagerung von DIDS oder SITS in DMSO über mehrere Tage bei Raumtemperatur bilden sich durch Reaktion der Isothiocyanat-Gruppen mit Amino-Gruppen Polymere (Fig. 6). In der Literatur wird die Polymerbildung vom DIDS bereits beschrieben (Schultz BD, Singh AK, Devor DC, Bridges RJ. Pharmacology of CFTR chloride channel activity. Physiol Rev. 1999 Jan;79(1 Suppl):S109-44. Review).

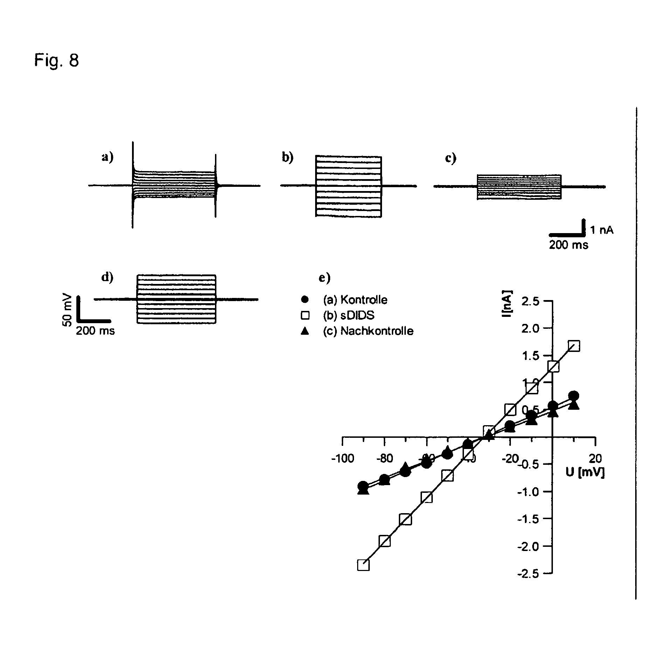

Bei den Experimenten zur Entwicklung des Test zeigte sich, dass die beobachtete Reaktion der roten Blutzellen sich nicht mit literaturbekannten Mechanismen erklären läßt. Die Blutzellreaktionen sind nur durch die Existenz eines bislang nicht literaturbekannten Kanalproteins erklärbar. Mittels patch-clamp Technik konnte an einer humanen Atemwegsepithelzelllinie (Calu-3) in der whole-cell-Konfiguration die reversible Aktivierbarkeit einer Leitfähigkeit (Kanalprotein) durch sDIDS beobachtet werden (Fig. 8).

Mit der whole-cell-Konfiguration der patch-clamp Technik wird der transmembranäre Strom einer Zelle bei vorgegebener Spannung gemessen. Die intrazelluläre Spannung wurde für jeweils 0,5 s auf Werte zwischen von -90 bis +10 mV gehalten und in 10 mV Schritten erhöht (8.d). Die bei diesem Spannungsverlauf auftretenden Strömen einer Zelle sind in 8a - 8c dargestellt. In 8a ist der Grundstrom einer unbehandelten Zelle gezeigt (Kontrolle). Durch Behandlung mit sDIDS steigt die Leitfahigkeit der Zellmembran dieser Zelle und damit der Strom (8b). Nachdem sDIDS aus dem Experiment entfernt wurde (Nachkontrolle), sinkt die Leitfähigkeit der Zellmembran dieser Zelle und damit der Strom (8c). In 8e ist der Strom in Abhängigkeit von der angelegten Spannung aufgetragen, wobei die Steigung der Kurve ein Maß für die Leitfähigkeit ist.

Das neue Kanalprotein lässt Ionen wie z. B.: Iodid, Bromid, Chlorid, Fluorid, Gluconat, Rhodanid und Taurin passieren. Zudem ist dieses Kanalprotein vom CFTR abhängig, da in Blutzellen von CF-Patienten mit sDIDS keine Leitfähigkeit von Ionen wie z. B.: Iodid, Bromid, Chlorid, Fluorid, Gluconat, Rhodanid und Taurin aktivierbar ist.

Diese Leitfähigkeit wird hier als "sDIDS activatable anion channel", kurz SDAC, bezeichnet und im folgenden SDAC genannt.

Die bisherigen Untersuchungen des Blutes von gesunden Personen, Mukoviszidose-Patienten und postnatal aus der Nabelschnur entnommenen Blutproben von Neugeborenen (Nabelschnur-Blut) bestätigen die einfache Durchführbarkeit und die gute Aussagekraft des erfindungsgemäßen Testverfahrens.

Das erfindungsgemäße Nachweisverfahren ist vorzugsweise mit Blutzellen einsetzbar. Besonders vorteilhaft zeigt sich der Einsatz von Retikulozyten und jungen Erythrozyten.

Retikulozyten sind die Vorläuferzellen der roten Blutkörperchen, der Erythrozyten. Sie werden im Knochenmark gebildet und ab dem Moment, in dem sie in den Blutkreislauf übertreten, Retikulozyten genannt. Sie verfügen über Zellorganellen und membranäre Transportsysteme. Nach ca. drei Tagen sind die Retikulozyten unter Verlust ihrer Zellorganellen und eines Teils ihrer membranären Transportsysteme zu jungen Erythrozyten gereift. Erythrozyten verweilen ca. 100 Tage im Blutkreislauf und verlieren im Laufe dieser Zeit immer mehr ihrer membranständigen Proteine. Da die Erythrozyten keinen Zellkern haben, ist die Neusynthese von Proteinen nicht möglich. Der Anteil der Retikulozyten im Vollblut beträgt 0,4 bis 2 % der Blutzellen beim Erwachsenen und bis zu 10 % beim Neugeborenen.

Das erfindungsgemäße Verfahren, die Volumenregulation von Blutzellen durch die Blockade transmembranärer Proteine zu beeinflussen oder durch Aktivierung CFTR-abhängiger Ionenkanäle eine starke Volumenaufnahme und Lyse nicht defekter Blutzellen zu verursachen, läßt sich für eine Vielzahl weiterer Untersuchungsmethoden zum Auffinden anderer Kanaldefekte, wie z.B. das Bartter-Syndrom, einsetzen. Beim Bartter-Syndrom handelt es sich um eine erbliche, nierenschädigende Erkrankung, die zu Lähmungen, Überwässerung und allgemeiner Schwächung des Körpers führen kann.

Das erfindungsgemäße Nachweisverfahren hat gegenüber den bislang eingesetzten Standardmethoden den Vorteil, dass es schnell, einfach, mit großem Probendurchsatz und somit kostengünstig einsetzbar ist. Das Untersuchungsmaterial, in Form von geringen Mengen Patientenblut, ist leicht verfügbar und mit den üblichen Einrichtungen und Gerätschaften eines Krankenhauslabors problemlos zu beschaffen und zu bearbeiten. Mit postnatal entnommenem Nabelblut kann ein Neugeborenen-Screening ohne invasiven Eingriff erfolgen.

Bevorzugtes Ausführungsbeispiel gemäß Verfahren 2:
Erfindungsgemäß werden zunächst Blutzellen aus Vollblut durch Zentrifugation gewonnen. Die Blutzellen werden einmalig mit isotoner Kochsalz-Lösung gewaschen und anschließend in einer Lösung, bestehend aus 155 mM KCI, 10 mM HEPES-Puffer (4-(2-Hydroxyethyl)piperazine-1-ethanesulfonic acid) (pH 7,4) und 100 mM GdCl₃, resuspendiert. Diese Lösung wird für 60 min unter Schütteln bei 37°C inkubiert und das freigesetzte Hämoglobin anschließend in der Lösung bestimmt. Die Retikulozyten und die jungen Erythrozyten aus dem Blut gesunder Probanden platzen und setzen ihr Hämoglobin frei; sie hämolysieren unter den gewählten Bedingungen. Die Hämolyse wird durch Sichtprüfung bestimmt und durch Absorptionsmessung bei einer Wellenlänge von 546 nm quantifiziert. Darüber hinaus kann auch eine Retikulozytenzählung im Ausstrich erfolgen. Dadurch wird der Test auch von jedem niedergelassenen Arzt durchführbar, der möglicherweise nicht über die Geräteausstattung eines Krankenhauslabors verfügt. ,

Durch den beschriebenen Einsatz des Gd³⁺ als Hemmstoff der ATP-Freisetzung steht eine preiswerte und einfach zu handhabende Chemikalie zur Verfügung. Der Test kann innerhalb einer Stunde ein zuverlässiges Ergebnis liefern. Der Arbeitszeit- und Materialaufwand ist in diesem Fall besonders gering.

Durch die in dem bevorzugten Ausführungsbeispiel des erfindungsgemäßen Verfahrens gewählten Testbedingungen strömen Chlorid-Ionen in die Blutzelle ein. Gesunde Blutzellen reagieren darauf mit einer autokrinen Signalkaskade, die durch CFTR-abhängige ATP-Freisetzung gestartet wird und zur Abgabe von Osmolyten führt. Influx und Efflux stehen im Gleichgewicht. Diese CFTR-abhängige ATP-Freisetzung ist durch die Zugabe der Chemikalie Gadoliniumchlorid (GdCl₃) hemmbar, was eine Akkumulation von Chlorid-Ionen in der Zelle zur Folge hat. Alternativ lassen sich auch andere Hemmstoffe dieser ATP-Freisetzung, z.B. Lanthan-Ionen (La³⁺) in Form von Lanthanchlorid (LaCl₃) und andere Hemmstoffe von deformations-aktivierbaren Kationenkanälen (stretch activatable cation channel, SAC) einsetzen. Der mit dem Ioneneinstrom verbundene Wassereinstrom führt zum Platzen der gesunden Blutzellen. Die Hämolyse kann photometrisch detektiert werden.

Bevorzugtes Ausführungsbeispiel gemäß Verfahren 3:
Das Verfahren gemäß Verfahren 2 wird analog zum Ausführungsbeispiel gemäß Verfahren 1 durchgeführt, nur werden die Blutzellen in einer Lösung, bestehend aus 155 mM KCI, 10 mM HEPES-Puffer (pH 7,4) und 100 µM sDIDS resuspendiert. Diese Lösung wird für 60 min unter Schütteln bei 37°C inkubiert und das freigesetzte Hämoglobin anschließend in der Lösung bestimmt.

Durch die in dem bevorzugten Ausführungsbeispiel des erfindungsgemäßen Verfahrens gewählten Testbedingungen strömen Chlorid-Ionen in nicht-defekten Blutzellen durch den durch sDIDS aktivierten Anionenkanal in die Blutzelle ein. Das führt zur Akkumulation von Chlorid-Ionen in der Blutzelle und der mit dem Ioneneinstrom verbundene Wassereinstrom führt zum Platzen der gesunden Blutzellen. Die Hämolyse kann photometrisch detektiert werden. Dieses Verfahren lässt sich auch mit 10 mM HEPES-gepufferter Lösung (pH 7,4) durchführen, die 155 mM Iodid, Bromid, Fluorid, Gluconat oder Rhodanid als Anion enthalten.

Die Anwendung des CF-Tests ist auch im Vollblut möglich. Dadurch gewinnt das Testverfahren weitere Einsatzmöglichkeiten, da es noch einfacher und schneller in der Durchführung wird.

Ein besonderer Vorteil des beschriebenen Tests besteht in der erstmalig gegebenen Möglichkeit, ein gesundes CFTR-Protein von einem mutierten kranken CFTR-Protein funktionell unterscheidbar zu machen und so auch heterozygote CFTR-Mutationen, die zu physiologischen Veränderungen führen, zuverlässig nachzuweisen. Im Vergleich zur genetischen Analyse ist das erfindungsgemäße Verfahren ein funktioneller Test, preiswerter und erlaubt aufgrund seiner schnellen Durchführbarkeit einen hohen Probendurchsatz, der besonders beim Screening von großer Bedeutung ist.

Durch das erfindungsgemäße Verfahren im Rahmen eines Neugeborenen-Screenings lassen sich beispielsweise die Folgeschäden und dadurch bedingt die Folgekosten der zystischen Fibrose einschränken und sinnvolle Risikoabschätzungen für die betroffenen Patienten vornehmen. Durch die frühestmögliche Diagnose können krankhafte Veränderungen der Lunge und des Verdauungstraktes vermieden oder zumindest in ihrer Ausprägung positiv beeinflußt werden, da gerade im ersten Lebensjahr schwere Komplikationen infolge intestinaler Störungen mit letalem Ausgang häufig zu verzeichnen sind. Im fortgeschrittenen Alter tritt der Tod vermehrt als Folge des Lungenbefalls, einschließlich Herzversagens, durch die Überlastung des Lungenkreislaufes ein. Darüber hinaus erlaubt eine rechtzeitig eingeleitete sinnvolle Therapie und die Vermeidung der Verabreichung ungeeigneter Antibiotika eine deutliche Verbesserung der Lebensqualität und in der Folge eine Erhöhung der Lebenserwartung der CF-Patienten.

In den Zeichungen zeigen:
- Fig. 1:: Statistische Auswertung der Experimente gemäß Anspruch 2.
- Fig. 2:: Statistische Auswertung der Experimente gemäß Anspruch 3.
- Fig. 3:: Massenspektrum der unsachgemäß gelagerten Lösung von DIDS in DMSO (0,1 M).
- Fig. 4:: partielle Hydrolyse von DIDS
- Fig. 5:: partielle Hydrolyse von SITS
- Fig. 6:: Polymerisierung von DIDS und 4-Amino-4'-Isothiocyanato-Stilben- 2,2'-Disulfonsäure
- Fig. 7:: 4-Amino-4'-Isothiocyanato-Dihydrostilben-2,2'-disulfonsäure, Dinatriumsalz
- Fig. 8:: Strom-Spannungs-Beziehung von Calu-3 Zellen, gemessen mit der patch-clamp-Technik in der whole-cell-Konfiguration.

## Patentansprüche

1. In vitro Verfahren zur Diagnose von CFTR-Defekten an einer Probe von Blutzellen umfassend folgende Schritte:
- Destabilisieren der CFTR abhängigen Volumenregulation und
- Diskriminieren zwischen CFTR-defekten und CFTR-intakten Zellen durch das Erfassen der Volumenregulation der CFTR-intakten Blutzellen durch die Darstellung der Zell-Lyse.

2. Verfahren zur Diagnose von CFTR-Defekten nach Anspruch 1, **gekennzeichnet durch** die Aktivierung (Öffnung) oder Hemmung eines CFTR-abhängigen Membrankanals.

3. Verfahren nach einem der vorherigen Ansprüche, **gekennzeichnet durch** eine Hämolyse der CFTR-intakten Blutzellen und Nicht-Hämolyse der CFTR-defekten Blutzellen.

4. Verfahren nach einem der vorherigen Ansprüche, **gekennzeichnet durch** ein lichtoptisches Verfahren zur Quantifizierung der Hämolyse.

5. Verfahren nach einem der Ansprüche 1 bis 3, **gekennzeichnet durch** eine Retikulozytenauszählung im Ausstrich zur Erfassung der Hämolyse.

6. Verfahren nach einem der vorherigen Ansprüche, **gekennzeichnet durch** die Verwendung eines Inhibitors der ATP-Freisetzung zur Hemmung der Volumenregulation.

7. Verfahren nach einem der vorherigen Ansprüche, **gekennzeichnet durch** die Verwendung eines Hemmstoffs für stretch activatable cation channels (SAC).

8. Verfahren nach Anspruch 7, **gekennzeichnet durch** die Verwendung von Gd³⁺ als Hemmstoff.

9. Verfahren nach einem der Ansprüche 1 bis 8, **gekennzeichnet durch** ein Mittel zur Aktivierung (Öffnung) des CFTR-abhängigen Ionenkanals SDAC.

10. Verfahren nach Anspruch 9, **gekennzeichnet durch** ein Stilbenderivat als Aktivierungsmittel.

11. Verfahren nach Anspruch 10, **gekennzeichnet durch** 4-Amino-4'-Isothiocyanato-Stilben-2,2'- Disulfonsäure davon als Aktivierungsmittel.

12. Verwendung eines Stilbenderivats zur Aktivierung des CFTR-abhängigen Ionenkanals SDAC in Blutzellen *in vitro*.

13. Verfahren nach einem der vorhergehenden Verfahrensansprüche 1-11, **gekennzeichnet durch** Ermitteln des Schweregrades der cystischen Fibrose anhand quantitativer Vergleiche.

14. Verfahren nach einem der vorherigen Ansprüche 9-10, 13, **gekennzeichnet durch** die Verwendung von 4-Amino-4'-Isothiocyanato-Dihydrostilben-2,2'-disulfonsäure als Aktivierungsmittel.

15. Diagnostik-Kit enthaltend 4-Amino-4'-Isothiocyanato-Dihydrostilben-2,2'-disulfonsäure.

16. Chemische Verbindung der allgemeinen Formel

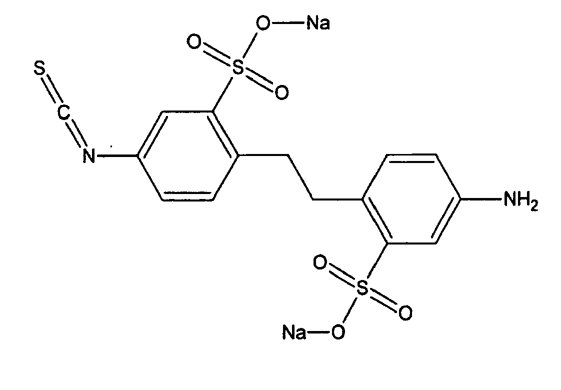

## Claims

1. *In vitro* method for diagnosing CFTR-defects on a sample of blood cells comprising the following steps:
- destabilizing the CFTR-dependent volume regulation
- discriminating between CFTR-defected and CFTR-intact cells by detecting the volume regulation of the CFTR-intact blood cells by depicting cell lysis.

2. Method for diagnosing CFTR-defects according to claim 1, **characterized by** the activation (opening) or inhibition of a CFTR-dependent membrane channel.

3. Method according to one of the proceeding claims, **characterized by** hemolysis of the CFTR-intact blood cells and a non-hemolysis of the CFTR-defective blood cells.

4. Method according to one of the proceeding claims, **characterized by** the use of a light-optical method for quantification of the hemolysis.

5. Method according to one of the claims 1 to 3, **characterized by** a reticulocyte count in the smear for detecting hemolysis.

6. Method according to one of the preceding claims, **characterized by** the use of an inhibitor of ATP release for inhibiting volume regulation.

7. Method according to one of the proceeding claims, wherein a substance from the group of inhibitors of stretch activatable cation channels (SAC) is used.

8. Method according to claim 7, **characterized by** the use of Gd³⁺ as an inhibitor.

9. Method according to one of the claims 1 to 8, **characterized by** an agent for activating (opening) the CFTR-dependent ion channel SDAC.

10. Method according to claim 9, **characterized by** a stilbene-derivative as an activator.

11. Method according to claim 10, **characterized by** 4-amino-4'-isothiocyanato-stilbene-2,2'-disulfonic acid thereof as activating agent.

12. Use of a stilbene-derivative for activating the CFTR-dependent ion channel SDAC in blood cells *in vitro.*

13. Method according to one of the proceeding claims 1 to 11, **characterized by** determining the degree of severity of cystic fibrosis by means of comparison.

14. Method according to one of the proceeding claims 9; 10; 13, **characterized by** the use of 4-amino-4'-isothiocyanato-stilbene-2,2'-disulfonic acid as an activating agent.

15. Diagnostic kit comprising 4-amino-4'-isothiocyanato-stilbene-2,2'-disulfonic acid.

16. Chemical compound of the general formula

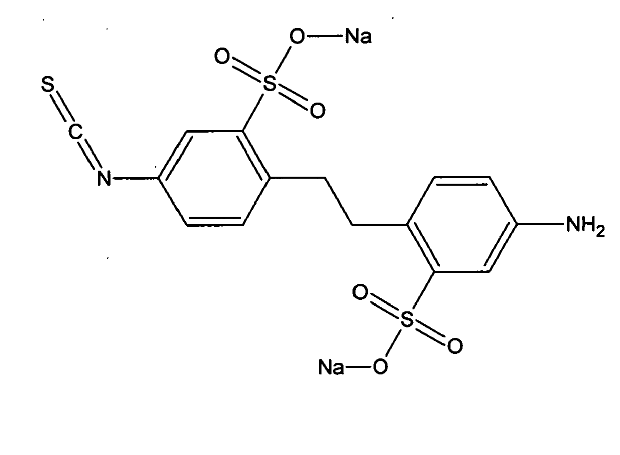

## Revendications

1. Procédé *in vitro* de diagnostic de défauts de la protéine CFTR dans un échantillon de cellules sanguines, comprenant les étapes suivantes :
- déstabilisation de la régulation du volume dépendant de la CFTR et
- discrimination entre cellules présentant un défaut de la CFTR et cellules à CFTR intacte par détection de la régulation du volume des cellules sanguines à CFTR intacte par la représentation de la lyse cellulaire.

2. Procédé de diagnostic de défauts de la CFTR selon la revendication 1, **caractérisé par** l'activation (ouverture) ou l'inhibition d'un canal membranaire dépendant de la CFTR.

3. Procédé selon l'une des revendications précédentes, **caractérisé par** une hémolyse des cellules sanguines à CFTR intacte et par une non-hémolyse des cellules sanguines présentant un défaut de la CFTR.

4. Procédé selon l'une des revendications précédentes, **caractérisé par** un procédé de quantification de l'hémolyse reposant sur l'optique de la lumière.

5. Procédé selon l'une des revendications 1 à 3, **caractérisé par** un décompte des réticulocytes dans le frottis pour saisir l'hémolyse.

6. Procédé selon l'une des revendications précédentes, **caractérisé par** l'utilisation d'un inhibiteur de la libération d'ATP pour inhiber la régulation du volume.

7. Procédé selon l'une des revendications précédentes, **caractérisé par** l'utilisation d'un inhibiteur des canaux SAC (stretch activatable cation channel, canal cationique activable par extension).

8. Procédé selon la revendication 7, **caractérisé par** l'utilisation de Gd³⁺ en tant qu'inhibiteur.

9. Procédé selon l'une des revendications 1 à 8, **caractérisé par** un agent d'activation (ouverture) du canal ionique CFTR-dépendant (SDAC).

10. Procédé selon la revendication 9, **caractérisé par** un dérivé stilbène en tant qu'agent d'activation.

11. Procédé selon la revendication 10, **caractérisé par** l'acide 4-amino-4'-isothiocyanatostilbène-2,2'-disulfonique en tant qu'agent d'activation.

12. Utilisation d'un dérivé de stilbène pour activer le canal ionique CFTR-dépendant SDAC dans des cellules sanguines *in vitro.*

13. Procédé selon l'une des revendications 1 à 11 précédentes, **caractérisé par** la détermination du degré de gravité de la mucoviscidose au moyen de comparaisons quantitatives.

14. Procédé selon l'une des revendications 9 à 10, 13, précédentes, **caractérisé par** l'utilisation d'acide 4-amino-4'-isothiocyanato-dihydrostilbène-2,2'-disulfonique en tant qu'agent d'activation.

15. Trousse de diagnostic contenant de l'acide 4-amino-4'-isothiocyanatodihydrostilbène-2,2'-disulfonique.

16. Composé chimique de formule générale
